(19) Europäisches Patentamt
European Patent Office
Office européen des brevets

(11) EP 2 011 865 B1

(12) # EUROPÄISCHE PATENTSCHRIFT

(45) Veröffentlichungstag und Bekanntmachung des Hinweises auf die Patenterteilung:
**12.08.2009 Patentblatt 2009/33**

(51) Int Cl.:
***C12N 11/00*** *(2006.01)* ***C08G 77/04*** *(2006.01)*

(21) Anmeldenummer: **08155937.9**

(22) Anmeldetag: **09.05.2008**

(54) **Enzympräparate**

Enzyme preparations

Préparations d'enzymes

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.07.2007 DE 102007031689**

(43) Veröffentlichungstag der Anmeldung:
**07.01.2009 Patentblatt 2009/02**

(73) Patentinhaber: **Evonik Goldschmidt GmbH**
**45127 Essen (DE)**

(72) Erfinder:
- **Thum, Oliver Dr.**
  **40880 Ratingen (DE)**
- **Ansorge-Schumacher, Marion Dr.**
  **52159 Roetgen (DE)**
- **Wiemann, Lars**
  **10559 Berlin (DE)**
- **Buthe, Andreas Dr.**
  **79636 Grenzach-Wyhlen (DE)**

(56) Entgegenhaltungen:
**EP-A- 0 231 093** **EP-A- 0 562 371**
**WO-A-03/106607**

- **BUTHE ET AL: "Generation of lipase-containing static emulsions in silicone spheres for synthesis in organic media" JOURNAL OF MOLECULAR CATALYSIS. B, ENZYMATIC, ELSEVIER, AMSTERDAM, NL, Bd. 35, Nr. 4-6, 1. September 2005 (2005-09-01), Seiten 93-99, XP005027013 ISSN: 1381-1177**
- **BRUNO L M ET AL: "Characterization of Mucor miehei lipase immobilized on polysiloxane-polyvinyl alcohol magnetic particles" WORLD JOURNAL OF MICROBIOLOGY AND BIOTECHNOLOGY, KLUWER ACADEMIC PUBLISHERS, DO, Bd. 21, Nr. 2, 1. März 2005 (2005-03-01), Seiten 189-192, XP019271520 ISSN: 1573-0972**
- **HOYOS P ET AL: "Highly efficient one pot dynamic kinetic resolution of benzoins with entrapped Pseudomonas stutzeri lipase" JOURNAL OF MOLECULAR CATALYSIS. B, ENZYMATIC, ELSEVIER, AMSTERDAM, NL, Bd. 52-53, 1. Juni 2008 (2008-06-01), Seiten 133-139, XP022586929 ISSN: 1381-1177 [gefunden am 2007-10-25]**

EP 2 011 865 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft neuartige Enzympräparate zur Verwendung als Biokatalysatoren.

Mikroorganismen und isolierte Enzyme finden verbreiteten Einsatz als Katalysator in der chemischen Industrie oder in der Lebensmittelherstellung. Eine Übersicht bietet beispielsweise: A. Liese, K. Seelbach, C. Wandrey, Industrial Biotransformations, Wiley-VCH: 2000, Weinheim, Deutschland.

Um einen ökonomischen Einsatz solcher Biokatalysatoren zu gewährleisten, müssen einige Bedingungen erfüllt sein: Der Biokatalysator muss unter den Reaktionsbedingungen hinreichend lange aktiv sein, er sollte nach Ende der Reaktion leicht abtrennbar und möglichst oft wieder verwendbar sein. Idealerweise sollten diese Bedingungen für eine möglichst große Breite an Reaktionsbedingungen (z. B. Temperaturbereich, Art der verwendeten Lösungsmittel, Drücke, etc.) erfüllt sein, um einen möglichst universellen Katalysator bereit zu stellen.

Um diese Bedingungen zu erfüllen, ist es üblicher Weise notwendig, die verwendeten Enzyme oder Enzyme enthaltenden Mikroorganismen zu immobilisieren.

Häufig werden die Enzyme oder Enzyme enthaltenden Mikroorganismen auf Trägern nichtkovalent immobilisiert, als Träger werden häufig Ionenaustauscherharze oder Polymerpartikel eingesetzt, die über geeignete Korngrößenverteilungen verfügen. Beispiele hierzu sind die Marktprodukte Novozym 435, Lipozym RM IM oder Lipozym TL IM der Firma Novozymes A/S, Bagsvaerd, Dänemark oder Amano PS, der Firma Amano, Japan. Bei diesen Beispielen handelt es sich um immobilisierte Lipasen, die breite Verwendung finden, da solche Immobilisate auch technisch nutzbare Aktivitäten in nichtwässrigen Systemen, also solchen, die keine oder nur organische Lösungsmittel enthalten, zeigen, wie z. B. in J. Chem. Soc., Chem. Comm. 1989, 934-935 beschrieben. Nachteilig an der Verwendung solcher Immobilisate ist allerdings zum einen die auftretende Desorption des Enzyms oder der Enzyme enthaltenden Mikroorganismen in Abhängigkeit von dem eingesetzten Reaktionssystem, beispielsweise bei Einsatz tensidischer Komponenten. Der mit einer solchen Desorption einhergehende Aktivitätsverlust ist in Vergleichsbeispiel 1 gezeigt. Darüber hinaus verfügen solche Präparate über eine ungenügende mechanische Stabilität, wodurch der Einsatz in einfachen Rührreaktoren nicht, oder nur unter Inkaufnahme deutlich eingeschränkter Wiederverwendbarkeit, möglich ist. Die mechanische Instabilität solcher Präparate wird in Vergleichsbeispiel 2 gezeigt.

Um den wiederholten Einsatz solcher Enzympräparate zu ermöglichen, müssen daher andere Reaktorkonzepte eingesetzt werden. Eur. J. Lipid Sci. Technol. 2003, 105, 601-607 beschreibt zum Beispiel den Einsatz eines Festbettreaktors zur Durchführung Lipase-katalysierter Veresterungen. Nachteilig an diesem Verfahren ist allerdings die Beschränkung auf niedrigviskose homogene Reaktionsmischungen, da hochviskose Mischungen oder Suspensionen nicht durch ein Festbett gefördert werden können.

K. Faber "Biotransformations in Organic Chemistry", Springer: 2000, Berlin, Deutschland, beschreibt auf Seite 384ff. die Verwendung von in Alginaten eingeschlossenen Enzymen. Allerdings weisen die so erhaltenen Präparate eine äußerst geringe mechanische Stabilität auf und zeigen nur sehr geringe Aktivität in nichtwässrigen Systemen.

Weiterhin wird die nachträgliche Quervernetzung von immobilisierten Enzymen mit reaktiven Substanzen, wie z. B. Glutardialdehyd beschrieben. Nachteil ist allerdings die meist deutlich reduzierte spezifische Aktivität der quervernetzen Präparate im Vergleich zu der Aktivität vor der Modifikation. Darüber hinaus liefert dieses Verfahren keinen Beitrag zur Verbesserung der mechanischen Stabilität.

Ebenfalls beschrieben wird dort die kovalente Immobilisierung von Enzymen auf reaktiven Trägern. Nachteilig hierbei ist, dass geeignete funktionelle Gruppen an der Oberfläche des Enzyms vorhanden sein müssen, die mit dem Träger reagieren können, weiterhin wird dadurch oftmals ein Verlust an Enzymaktivität erzielt.

J. Am. Chem. Soc. 1999, 121, 9487-9496 beschreibt den Einschluss von Enzymen in Siloxanmatrizes, sogenannte Sol-Gele. Nachteilig an Sol-Gel Präparationen ist die niedrige Korngrößenverteilung, die ein effizientes Abfiltrieren erschwert, die mangelnde mechanische Stabilität, auftretende Desorption, der Einsatz giftiger Reaktanden (zur Toxizität von TEOS siehe z. B. Nippon Sanso Giho 1990, 9, 68-72 und Archives of Toxicology 1994, 68, 277-283; zur Giftigkeit von TMOS siehe z. B. Fundamental and Applied Toxicology 1989, 13, 285-295; zur Herstellung nicht-giftiger Sol-Gele sind aufwändige Schritte nötig, z. B. Lagerung über 6 Monate oder thermische Behandlung bei 350 °C, wie in Polimery w Medycynie 2000, 30, 45-54 beschrieben. sowie das sehr stark vom verwendeten Lösungsmittel abhängige Quellverhalten, welches eine universelle Verwendung in verschiedenen Reaktionssystemen (wässrig und nichtwässrig) nicht zulässt. J. Sol Gel Sci. Technol. 2003, 26, 1183-1187 zeigt beispielhaft die Lösungsmittelabhängigkeit der beobachteten Enzymaktivität und damit das Nichterfüllen des Anspruchs der breiten Einsetzbarkeit.

Landbauforschung Völkenrode, 2002, Sonderheft 241, 41-46 beschreibt Sol-Gel Präparate, bei denen Enzyme zunächst auf "feine" Siliconpartikel immobilisiert und anschließend in ein Sol-Gel verkapselt werden. So wird das Problem der mechanischen Stabilität teilweise gelöst, die beschriebenen Experimente zeigen aber, dass hinreichende Aktivitäten nur in ausgewählten Lösungsmitteln erreicht werden, die Verwendung im lösungsmittelfreien System wird gar nicht beschrieben. Darüber hinaus fallen die Präparationen nicht in einer direkt verwendbaren Form an, sondern müssen erst auf passende Große geschnitten werden, was im technischen Maßstab kaum zu realisieren ist.

J. Mol. Catal. B, 2005, 35, 93-99 beschreibt die Immobilisierung von Enzymen durch Einschluss wässriger

Enzymlösungen in mechanisch stabile Siliconsphären, sogenannte statische Emulsionen. Die erhaltenen spezifischen Aktivitäten der Präparate sind aber mit bis zu 33 U/g deutlich zu niedrig im Vergleich zu den oben beschriebenen Immobilisaten auf inerten Trägern, wo leicht spezifische Aktivitäten von über 1000 U/g erzielt werden können (U = Unit oder $\mu$mol/min).

WO 03/106607 A1 beschreibt ebenfalls solche statischen Emulsionen, allerdings wird ausschließlich die Verwendung in wässrigen Systemen beschrieben, die Anwendung ist eine Waschmittelzusammensetzung, also keine Biokatalyse, und die erhaltenen Korngrößen sind mit ca. 10 $\mu$m zu klein für ein effizientes Abfiltrieren aus organischen Reaktionsmischungen.

Es besteht daher weiterhin Bedarf an Methoden zur Enzymimmobilisierung, welche die Nachteile des Standes der Technik überwinden, um bisher nicht realisierbare biokatalytische Verfahren umzusetzen.

Aufgabe der vorliegenden Erfindung war daher die Bereitstellung von Enzympräparaten, die einen oder mehrere der Nachteile der Präparate des Standes der Technik nicht aufweisen. Insbesondere sollten Enzympräparate bereitgestellt werden, die eine hohe Stabilität gegenüber mechanischen Kräften und gegenüber Desorption aufweisen und dabei vorzugsweise über spezifische Aktivitäten in verschiedenen wässrigen und nichtwässrigen Reaktionsmischungen verfügen, die hoch genug sind, um einen technischen Einsatz zu ermöglichen. Vorzugsweise sollten die Enzympräparate von ihrer Korngrößenverteilung her geeignet sein, einfach vom Reaktionssystem abgetrennt und wiederverwendet werden zu können.

Weitere nicht explizit genannte Aufgaben ergeben sich aus dem Kontext der nachfolgenden Beschreibung, der Beispiele sowie der Ansprüche.

Überraschender Weise wurde gefunden, dass diese Aufgabe durch Enzympräparate gelöst wird, die durch Immobilisieren von Enzymen oder Enzyme enthaltenden Mikroorganismen auf einem inerten Träger und anschließendes Überziehen mit einer durch Hydrosilylierung gewonnenen Siliconbeschichtung erhalten werden.

Gegenstand der vorliegenden Erfindung sind daher Enzympräparate, die dadurch erhältlich sind, dass Enzymimmobilisate, die Enzyme oder Enzyme enthaltende Mikroorganismen auf einen inerten Träger immobilisiert aufweisen, mit einer durch Hydrosilylierung gewonnenen Siliconbeschichtung versehen werden sowie deren Verwendung als industrieller Biokatalysator.

Außerdem ist Gegenstand der vorliegenden Erfindung ein Verfahren zur Herstellung der erfindungsgemäßen Enzympräparate, welches dadurch gekennzeichnet ist, dass Enzymimmobilisate, die Enzyme oder Enzyme enthaltende Mikroorganismen auf einen inerten Träger immobilisiert aufweisen, mit einer durch Hydrosilylierung gewonnenen Siliconbeschichtung versehen werden.

Die erfindungsgemäßen Enzympräparate haben den Vorteil, dass sie eine hohe Stabilität gegenüber mechanischen Kräften und gegenüber Desorption aufweisen. Trotz dieser verbesserten Eigenschaften weisen die erfindungsgemäßen Enzympräparate spezifische Aktivitäten in verschiedenen wässrigen (z. B. in der Hydrolyse von Tributyrin) und nichtwässrigen Reaktionsmischungen (z. B. in der lösungsmittelfreien Synthese von Propyllaurat) auf, die hoch genug sind, um einen technischen Einsatz zu ermöglichen. Die erfindungsgemäßen Enzympräparate weisen außerdem den Vorteil auf, dass durch die Wahl des Trägermaterials und der damit einhergehenden Korngrößenverteilung die Partikelgröße so eingestellt werden kann, dass eine einfache Abtrennung der Enzympräparate vom Reaktionssystem und somit auch die Wiederverwendung der Enzympräparate möglich ist.

Die erfindungsgemäßen Enzympräparate sowie ein Verfahren zu deren Herstellung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen.

Die erfindungsgemäßen Enzympräparate zeichnen sich dadurch aus, dass sie erhältlich sind, indem Enzymimmobilisate, die Enzyme oder Enzyme enthaltende Mikroorganismen auf einen inerten Träger immobilisiert aufweisen, mit einer Siliconbeschichtung versehen werden, die durch Hydrosilylierung gewonnen wird.

Zur Herstellung der Enzymimmobilisate können ganze Zellen, ruhende Zellen, gereinigte Enzyme oder Zellextrakte, die die entsprechenden Enzyme enthalten oder Mischungen davon eingesetzt werden. Bevorzugt eingesetzt werden hydrolytische Enzyme, z. B. Lipasen, Esterasen oder Proteasen, wie beispielsweise Lipasen aus Candida rugosa, Candida antarctica, Pseudomonas sp., Thermomyces langosiosus, Schweinepankreas, Mucor miehei, Alcaligines sp., Cholesterolesterase aus Candida rugosa, Esterase aus der Schweineleber, besonders bevorzugt Lipasen, eingesetzt. Demgemäß weisen die Enzymimmobilisate vorzugsweise Enzyme aus der Klasse der Hydrolasen, bevorzugt

Lipasen, auf.

Als inerte Träger können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 10 bis 5000 μm, bevorzugt von 50 μm bis 2000 μm aufweisen. Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylenperepthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica, Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Polyvinylstyrol, Polymethacrylat oder Polyacrylat.

Die Immobilisierung auf den Partikeln kann erfindungsgemäß kovalent oder nichtkovalent, bevorzugt nichtkovalent erfolgen. Für die nichtkovalente Immobilisierung kann der Träger z. B. mit einer wässrigen Enzymlösung, die gegebenenfalls weitere Bestandteile, wie z. B. anorganische Salze oder Detergenzien, enthalten kann, inkubiert bzw. imprägniert werden. Diese Inkubation/Imprägnierung kann z. B. bei Temperaturen zwischen 0 °C und 50 °C, bevorzugt zwischen 0 °C und 40 °C durchgeführt werden. Vorzugsweise erfolgt die Inkubation/Imprägnierung über einen Zeitraum von wenigen Minuten bis zu einigen Stunden. Der Fortschritt der Inkubation kann durch Bestimmung der Konzentration des Enzyms in der Lösung mit den gängigen Verfahren zur Proteinbestimmung erfolgen. Nach Erreichen des gewünschten Immobilisierungsgrads kann der Träger vorzugsweise mit Wasser gewaschen und, wenn gewünscht, getrocknet werden. Ein so erhaltenes Enzymimmobilisat kann anschließend erfindungsgemäß mit einer Siliconbeschichtung versehen werden.

Erfindungsgemäß können aber auch Enzymimmobilisate eingesetzt werden, die kommerziell erhältlich sind, beispielsweise Novozym 435, Lipozym RM IM oder Lipozym TL IM der Firma Novozymes A/S, Bagsvaerd, Dänemark oder Amano PS, der Firma Amano, Japan.

Die Siliconbeschichtung wird erfindungsgemäß durch Hydrosilylierung erhalten. Dazu werden vorzugsweise Si-H-funktionelle Polysiloxane in Gegenwart von Katalysatoren, vorzugsweise von Übergangsmetallkatalysatoren, mit organomodifizierten Polysiloxanen umgesetzt, die über mindestens eine endständige, vorzugsweise zumindest zwei Kohlenstoff-Kohlenstoff Doppelbindung(en) verfügen.

Als Si-H-funktionelle Polysiloxane werden vorzugsweise SiH-Polysiloxane der allgemeinen Formel I eingesetzt,

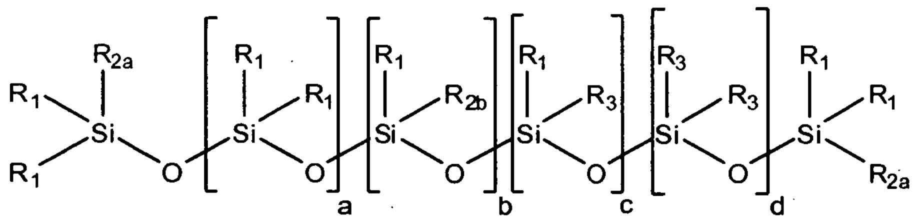

(I)

wobei

N = a + b + c + d + 2 = 3 bis 850, vorzugsweise 6 bis 160,

a = 1 bis 800, vorzugsweise 2 bis 150,

b = 0 bis 400, vorzugsweise 2 bis 75,

c = 0 bis 10, vorzugsweise 0,

d = 0 bis 10, vorzugsweise 0,

$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;

$R_{2a}$ unabhängig voneinander Wasserstoff oder $R_1$;

$R_{2b}$ unabhängig voneinander Wasserstoff oder $R_1$;
$R_3$ unabhängig voneinander gleiche oder verschiedene Reste der allgemeinen Formel Ia

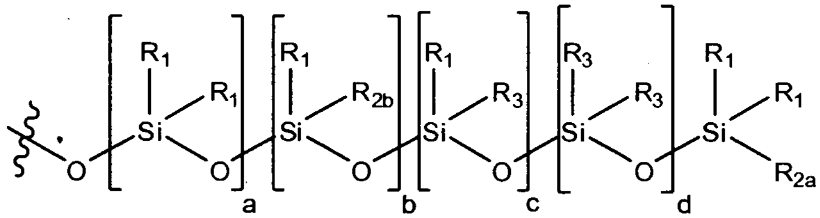

(Ia)

sind, wobei

N = a + b + c + d + 2 = 3 bis 850, vorzugsweise 6 bis 160,
a = 1 bis 800, vorzugsweise 2 bis 150,
b = 0 bis 400, vorzugsweise 2 bis 75,
c = 0 bis 10, vorzugsweise 0,
d = 0 bis 10, vorzugsweise 0,
$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
$R_{2a}$ unabhängig voneinander Wasserstoff oder $R_1$;
$R_{2b}$ unabhängig voneinander Wasserstoff oder $R_1$;
$R_3$ unabhängig voneinander gleiche oder verschiedene Reste der Formel Ia oder ein Rest $R_1$.

Bevorzugt wird als SiH-funktionelles Polysiloxan ein Polysiloxan der allgemeinen Formel I

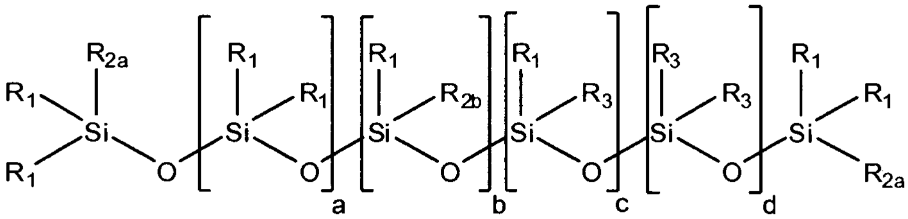

(I)

eingesetzt, mit

N = a + b + c + d + 2 = 6 bis 160,
a = 2 bis 150,
b = 2 bis 75,
c = 0,
d - 0,
$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
$R_{2a}$ unabhängig voneinander Wasserstoff oder $R_1$;
$R_{2b}$ unabhängig voneinander Wasserstoff oder $R_1$.

Es ist dem Fachmann geläufig, dass die Verbindungen in Form eines Gemisches mit einer im Wesentlichen durch statistische Gesetze geregelten Verteilung vorliegen bzw. vorliegen können. Die Werte für die Indices a, b, c und d stellen deshalb üblicherweise Mittelwerte dar.

Erfindungsgemäß werden als olefinische Reaktionspartner, also als endständige Kohlenstoff-Kohlenstoff Doppelbindung enthaltende Polysiloxane vorzugsweise Polysiloxane der allgemeinen Formel II:

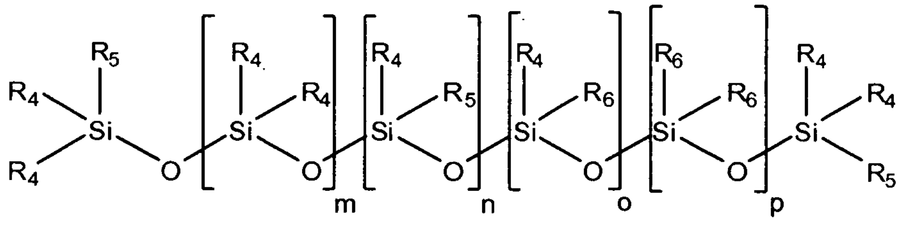

(II)

verwendet, wobei

N = m + n + o + p + 2 = 3 bis 1000, vorzugsweise 10 bis 600,
m = 1 bis 800, vorzugsweise 2 bis 600,
n = 0 bis 20, vorzugsweise 0 bis 10, bevorzugt 0,
o = 0 bis 10, vorzugsweise 0,
p = 0 bis 10, vorzugsweise 0,
$R_4$ unabhängig voneinander gleich oder verschieden und aus folgender Gruppe sind: gesättigte oder ungesättigte, gegebenenfalls verzweigte Alkylgruppen mit 1 bis 30 C- Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
$R_5$ unabhängig voneinander ein terminal ungesättigter Alkylrest, bevorzugt Vinyl, oder ein Alkoxylrest, vorzugsweise mit 3 bis 20 C-Atomen, oder $R_4$;
$R_6$ unabhängig voneinander gleiche oder verschiedene Reste der allgemeinen Formel IIa

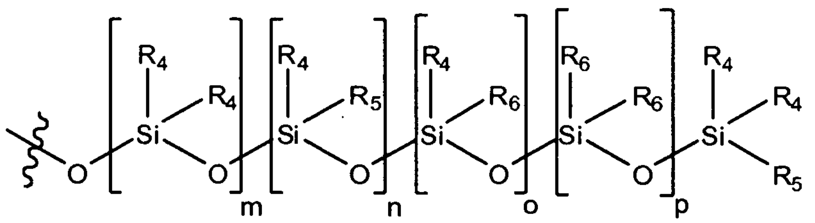

(IIa)

sind, wobei
N = m + n + o + p + 2 = 3 bis 1000, vorzugsweise 10 bis 600,
m = 1 bis 800, vorzugsweise 2 bis 600,
n = 0 bis 20, vorzugsweise 0 bis 10, bevorzugt 0,
o = 0 bis 10, vorzugsweise 0,
p = 0 bis 10, vorzugsweise 0,
$R_4$ unabhängig voneinander gleich oder verschieden und aus folgender Gruppe sind: gesättigte oder ungesättigte, gegebenenfalls verzweigte Alkylgruppen mit 1 bis 30 C- Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
$R_5$ unabhängig voneinander ein terminal ungesättigter Alkylrest, bevorzugt Vinyl, oder ein Alkoxylrest, vorzugsweise mit 3 bis 20 C-Atomen, oder $R_4$;
$R_6$ unabhängig voneinander gleiche oder verschiedene Reste der allgemeinen Formel IIa oder Reste $R_4$.

Als endständige Kohlenstoff-Kohlenstoff Doppelbindung enthaltende Polysiloxane werden bevorzugt Polysiloxane der allgemeinen Formel II:

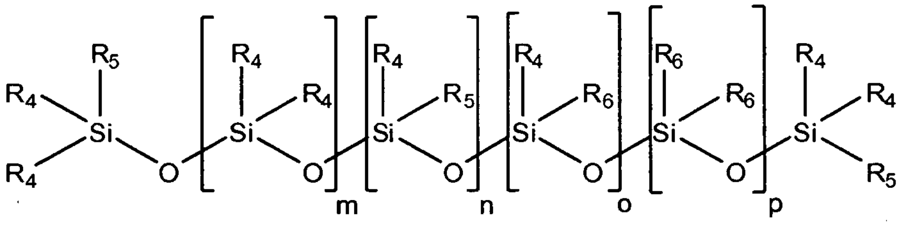

(II)

verwendet, mit

N = m + n + o + p + 2 = 10 bis 600,
m = 2 bis 600,
n = 0,
o - 0,
p - 0,
$R_4$ unabhängig voneinander gleich oder verschieden und aus folgender Gruppe sind: gesättigte oder ungesättigte, gegebenenfalls verzweigte Alkylgruppen mit 1 bis 30 C- Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl;
$R_5$ unabhängig voneinander ein terminal ungesättigter Alkylrest, bevorzugt Vinyl, oder ein Alkoxylrest, vorzugsweise mit 3 bis 20 C-Atomen.

Es ist dem Fachmann geläufig, dass die Verbindungen der Formel II in Form eines Gemisches mit einer im Wesentlichen durch statistische Gesetze geregelten Verteilung vorliegen bzw. vorliegen können. Die Werte für die Indices m, n, o, und p stellen deshalb üblicherweise Mittelwerte dar.

Die Hydrosilylierung kann nach etablierten Methoden in Gegenwart eines Katalysators durchgeführt werden. Dabei können beispielsweise Katalysatoren verwendet werden, die üblicherweise für Hydrosilylierungen eingesetzt werden, wie beispielsweise Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierte Derivate. Bevorzugt wird die Hydrosilylierung in Gegenwart von Pt-Katalysatoren wie Cis-Platin oder Karstedt-Katalysator [Tris(divinyltetramethyldisiloxan)bis-platin] durchgeführt.

Vorzugsweise beträgt die eingesetzte Menge an Katalysator $10^{-7}$ bis $10^{-1}$ mol pro mol Olefin bzw. pro mol endständiger Kohlenstoff-Kohlenstoff Doppelbindung, bevorzugt 1 bis 100 ppm. Die Hydrosilylierung wird vorzugsweise bei Temperaturen von 0 und 200 °C, bevorzugt von 20 bis 120 °C, durchgeführt.

Die Hydrosilylierung kann in Gegenwart oder in Abwesenheit von Lösungsmittel durchgeführt werden. Generell sind Lösungsmittel für die Durchführung der Reaktion nicht nötig. Die Reaktion kann jedoch in geeigneten Lösungsmitteln, wie beispielsweise aliphatischen oder aromatischen Kohlenwasserstoffen, cyclischen Oligosiloxanen, Alkoholen oder Estern, durchgeführt werden. Geeignete Lösungsmittel sind insbesondere Cyclohexan oder Toluol.

Erfindungsgemäß werden bezogen auf die Masse des eingesetzten Trägers vorzugsweise 1 bis 500 Massen-%, bevorzugt 10 bis 200 Massen-%, besonders bevorzugt 20 bis 150 Massen-% an Siloxankompenenten eingesetzt. Die Siloxankomponenten setzen sich insbesondere aus der Summe der Verbindungen der Formel I und II bzw. aus deren Reaktionsprodukten zusammen.

Die Hydrosilylierung kann unter Verwendung der verschiedensten Verhältnisse der Verbindungen der Formeln I zu Verbindungen der Formeln II durchgeführt werden. Vorzugsweise erfolgt die Hydrosilylierung bei einem molaren Verhältnis bezogen auf die reaktiven Gruppen von 1 : 10 bis 10 zu 1, bevorzugt von 1 : 5 bis 5 : 1, besonders bevorzugt von 1 : 1,1 bis 1,1 : 1 und ganz besonders bevorzugt von 1 : 1. Durch Wahl der eingesetzten Verbindungen der allgemeinen Formel I und II und Veränderung ihrer Mischungsverhältnisse lassen sich die Eigenschaften der Siliconbeschichtung Maßschneidern in Hinblick auf Durchlässigkeit für Substrate und andere Reaktionseigenschaften. Durch Wahl des Gewichtsverhältnisses von Siliconkomponenten zu Enzymimmobilisaten lassen sich die Schichtdicken der Siliconbeschichtung variieren und an entsprechende Anforderungen angleichen.

Die erfindungsgemäße Siliconbeschichtung, hergestellt durch Hydrosilylierung, kann dadurch erhalten werden, dass die Hydrosilylierung in Gegenwart der Enzymimmobilisate durchgeführt wird. Es ist aber auch möglich die Überzüge dadurch zu erhalten, dass ein durch Hydrosilylierung erhaltenes Siloxan nachträglich auf die Enzymimmobilisate aufgebracht wird. Dies kann z. B. so erfolgen, dass die Enzymimmobilisate mit einer Lösung des Siloxans, z. B. einer Lösung des Siloxans in einem organischen Lösungsmittel, insbesondere Cyclohexan oder Toluol, behandelt wird. Anschließend kann das Lösungsmittel z. B. durch Abdampfen entfernt werden. Die Konzentration an Siloxan in einer solchen Lösung beträgt vorzugsweise 10 bis 100 Massen-%, bevorzugt 30 bis 100 Massen-%. Vorzugsweise wird die erfindungsgemäße Siliconbeschichtung aber dadurch erhalten, dass die Hydrosilylierung in Gegenwart der Enzymimmobilisate durchgeführt wird.

Die erfindungsgemäßen Enzympräparate werden vorzugsweise hergestellt durch das nachfolgend beschriebene erfindungsgemäße Verfahren. Dieses Verfahren zur Herstellung von Enzympräparaten zeichnet sich dadurch aus, dass Enzymimmobilisate, die Enzyme oder Enzyme enthaltende Mikroorganismen auf einen inerten Träger immobilisiert aufweisen, mit einer durch Hydrosilylierung gewonnenen Siliconbeschichtung versehen werden.

Vorzugsweise wird das erfindungsgemäße Verfahren so durchgeführt, dass die Enzymimmobilisate dadurch mit einer Siliconbeschichtung versehen werden, dass die Enzymimmobilisate mit einer Reaktionsmischung, die SiHfunktionale Polysiloxane, endständige Kohlenstoff-Kohlenstoff Doppelbindungen enthaltende Polysiloxane sowie einen die Hydrosilylierung katalysierenden Katalysator aufweist, unter Hydrosilylierungsbedingungen in Kontakt gebracht werden. Insbesondere kann das Verfahren so durchgeführt werden, dass in Gegenwart von Enzymimmobilisaten, die Enzyme oder Enzyme enthaltende Mikroorganismen auf einen inerten Träger immobilisiert aufweisen, eine Hydrosilylierungsreaktion durchgeführt wird. Durch das bei der Hydrosilylierung entstehende Silicon kann das Enzymimmobilisat mit einer Siliconbeschichtung versehen werden.

Die Hydrosilylierung kann auf eine dem Fachmann bekannte Weise durchgeführt werden. Vorzugsweise wird die Hydrosilylierung unter Verwendung der oben genannten Parameter/Einsatzstoffe/Katalysatoren durchgeführt.

In einer bevorzugten Ausführungsform des erfindungsgemäßen Verfahrens wird eine bestimmte Menge Enzymimmobilisat mit einer Mischung (Reaktionsmischung) der Siliconreagentien (Verbindungen der Formeln I und II plus Katalysator) versetzt, beispielsweise durch Zugabe von einer Mischung enthaltend Verbindungen der allgemeinen Formel I und der allgemeinen Formel II sowie Karstedt-Katalysator. So kann z. B. zu 1 g eines Enzymimmobilisats eine Mischung von Verbindungen der Formel I und II im molaren Mischungsverhältnis von 10:1 bis 1:10, sowie Karstedt-Kat, bspw. 50 ppm bezogen auf die vorhandene Menge an Siliconkomponenten gegeben werden. Zwecks Optimierung der Beschichtungseigenschaften kann es vorteilhaft sein, die Siliconkomponenten inklusive des Katalysators vor der Zugabe in einem Lösungsmittel, beispielsweise Cyclohexan, Toluol oder einem anderen organischen Lösungsmitteln zu lösen und dann die Lösung zu dem Enzymimmobilisat zu geben. Wird beispielsweise Cyclohexan als Lösungmittel verwendet, hat es sich als vorteilhaft erwiesen, nach Zugabe der Lösung zu den Enzymimmobilisaten diese Mischung für ca. 15 bis 30 min. stark zu dispergieren, z. B. durch einen Vortexer (Ika, Stufe 9), bis ein Gros des Cyclohexans abgedampft ist. Anschließend werden die erhaltenen Enzympräparate im Trockenschrank bei 50 °C beispielsweise für 12 Stunden getrocknet, bzw. gehärtet. Durch Veränderung der Mischungsverhältnisse der Verbindungen der allgemeinen Formel I und II können die Eigenschaften des Siliconcoatings problemlos variiert und an entsprechende Anforderungen angeglichen werden.

Eine weitere Ausführungsform des erfindungsgemäßen Verfahrens unterscheidet sich von der vorgenannten Ausführungform dadurch, dass die zu beschichtenden Enzymimmobilisate in die gewünschte Reaktionsmischung eingetaucht, anschließend aus der Reaktionsmischung entfernt und getrocknet werden. Das Entfernen kann z. B. unter Verwendung eines Siebes, das die Enzymimmobilisat-Partikel zurückhält erfolgen. Die Eintauchdauer beträgt vorzugsweise von 1 bis 10 Minuten. Das Trocknen kann in einem herkömmlichen Trockenschrank erfolgen. Vorzugsweise erfolgt die Trocknung/Härtung bei einer Temperatur von 20 °C bis 80 °C, bevorzugt bei 40 °C bis 60 °C, besonders bevorzugt bei ca. 50 °C.

In einer weiteren Ausführungsform des erfindungsgemäßen Verfahrens, die insbesondere zur Durchführung im technischen Maßstab geeignet ist, wird die Hydrosilylierung unter Verwendung einer Pelletiertellereinheit (bspw. der Fa. Erweka oder Eirich) durchgeführt. Dabei wird eine definierte Menge an Enzymimmobilisat-Partikeln in die sogenannte Tellereinheit gegeben und gerührt. Anschließend wird entweder die Mischung, enthaltend Verbindungen der Formeln I und II, sowie Katalysator sowie ggf. Lösungsmittel zugegeben oder aber vorzugsweise wird, unter Verwendung einer Zweistoffdüse (z. B. der Firma Schlick oder andere), in welcher unter Druck (bspw. Stickstoff oder synthetische Luft) die Mischung bzw. die Komponenten in Form eines feinen Tröpfchennebels appliziert, um eine möglichst homogene Verteilung auf den Partikeln zu gewährleisten. Die Partikel werden nach einer längeren Beschichtungszeit, wie oben beschrieben entnommen und für einige Stunden bei einer Temperatur von 20 °C bis 80 °C, bevorzugt von 40 °C bis 60 °C, besonders bevorzugt von 50 °C im Trockenschrank getrocknet, bzw. gehärtet, und können anschließend bis zur weiteren Verwendung bei Raumtemperatur gelagert werden.

In einer weiteren Ausführungsform können die Partikel in einem Wirbelschichtreaktor (bspw. Fa. Erwerka) generiert werden, in dem Partikel und die Reaktionsmischung unter starker Dispergierung in entsprechenden Mischungsverhältnissen appliziert werden.

Die erfindungsgemäßen Enzympräparate können z. B. als Biokatalysatoren, insbesondere als industrielle Biokatalysatoren verwendet werden.

Die vorliegende Erfindung wird an Hand der Abbildungen Fig. 1 und 2 näher erläutert, ohne darauf beschränkt zu sein.

Die Abbildungen Fig. 1 und Fig. 2 zeigen Aufnahmen von gerührten Suspensionen in einem 100 mL Becherglas mit einem Durchmesser des Bodens von 4 cm. Die Suspensionen sind wie in Beispiel 2 beschrieben hergestellt worden. Der Abbildung Fig. 1 kann entnommen werden, dass die gerührte Suspension basierend auf unbehandeltem NZ435 von feinen Partikeln getrübt ist. Die gerührte Suspension basierend auf erfindungsgemäß behandeltem NZ435 ist dagegen klar, weist also keine Partikel bzw. zumindest keine Partikel in einer sichtbaren Größe auf (Fig. 2).

Die nachfolgenden Beispiele sollen die vorliegende Erfindung näher erläutern, ohne den Schutzbereich einzuschränken, der sich aus der Beschreibung und den Patentansprüchen ergibt.

Beispiele

Materialien und Methoden:

Novozym 435 (NZ435) ist ein kommerzielles Enzymimmobilisat der Firma Novozymes A/S, Bagsvaerd/Dänemark, einer auf einem Polymethyacrylat durch Adsorption immobilisierten Lipase B aus *C. antarctica.*

Hydrolytische Aktivität (Tributyrinhydrolyse in wässrigem Medium):

Die hydrolytische Aktivität wurde mit der sogenannten pH-Stat.-Methode bestimmt, hierbei wird die in der Hydrolyse freigesetzte Säure gegen eine Base titriert, so dass der pH-Wert der Lösung konstant gehalten wird. Aus der Zeitabhängigkeit des Verbrauchs an Base läßt sich die freigesetzte Säure, und damit die Enzymaktivität quantifizieren. Exemplarische Durchführung: Es wurden 10-20 mg katalytisch aktive Partikel zu 25 mL Tris-HCl-Puffer (1 mM, pH 7,5; enthält zusätzlich 0,1 mM NaCl und $CaCl_2$) und 500 µL Tributyrin gegeben. Die hydrolytische Aktivität wurde am Autotitrator (Tritroline alpha, Fa. Schott) über die Menge der zutitrierten Base (50 mM NaOH) quantifiziert.

Hydrolytische Aktivität (Valeriansäureethylester in wässrigem Medium):

Analog zur Bestimmung der Hydrolysaktivität am Beispiel von Tributyrin kann auch Valeriansäureethylester verwendet werden. Exemplarische Durchführung: Es wurden 10-20 mg katalytisch aktive Partikel zu 25 mL Phosphat-Puffer (1 mM, pH 8,0) und 500 µL Valeriansäureethylester gegeben. Die hydrolytische Aktivität wurde am Autotitrator (Tritroline alpha, Fa. Schott) über die Menge der zutitrierten Base (10 mM NaOH) quantifiziert.

Syntheseaktivität in PLU Units (Propyllauratsyntheseaktivität in lösungsmittelfreiem System):

Es wurden 10 mg katalytisch aktive Partikel in 5 mL äquimolare Substratlösung (Laurinsäure und 1-Propanol) gegeben und bei 60 °C schüttelnd bzw. rührend inkubiert. Proben ($V_{probe}$: 50 µL) wurden über 25 min alle 5 min gezogen und in 950 µL Decan (Interner Standard: 4 mM Dodecan) überführt. Die Bestimmung der PLUs erfolgte anhand der initialen Produktbildungsraten. Der Nachweis von Propyllaurat (Retentionszeit: 9.791 min) erfolgte gaschromatografisch (Shimadzu 2010, Säule BTX Fa. SGE; Länge 25 m , I.D. 0,22 µm; Film: 0,25 µM; Detektortyp: FID bei 300 °C; Injektortemperatur 275 °C und Injektionsvolumen 1 µL, Splitratio 35.0; Trägergasdruck (Helium) 150 kpa; Temperaturprogramm: Anfangstemperatur 60 °C hold 1,5 min, Temperaturanstieg 20 °C/min, Endtemperatur 250 °C hold 2,5 min) .

Bestimmung der Laccaseaktivität:

Zur Bestimmung der Laccaseaktivität werden katalytisch aktive Partikel (native oder immobilisierte Laccase) in 19 mL Kaliumphosphatpuffer (100 mM, pH 6, 37 °C) mit 1 mL ABTS-Lsg. (Ready-to-use-Lösung, 1,8 mM, erhältlich von Sigma-Aldrich) überführt und die Zunahme der Extinktion photospektrometrisch bei 405 nm gemessen. Die Laccaseaktivität soll über eine Zeitspanne von 20 min verfolgt werden. Die Proben werden im Abstand von 5 min genommen.

Die Aktivität lässt sich folgendermaßen bestimmen:

$$Aktivität = \frac{\Delta Ext._{405} \cdot V_{gesamt}}{\Delta t \cdot \varepsilon \cdot d \cdot V_{Probe}}$$

| | |
|---|---|
| $\Delta$ Ext. $_{405}$ | Änderung der Extinktion in Abhängigkeit von der Zeit |
| $V_{gesamt}$ | Gesamtvolumen in des Reaktionsansatzes [20 mL] |
| $V_{Probe}$ | Volumen der Probe [2 mL] |
| $\Delta t$ | Änderung der Zeit [min] |
| $\varepsilon$ | Extinktionskoeffizient für ABTS bei 405 nm [43,2 mL $\mu mol^{-1}$ $cm^{-1}$] |
| d | Schichtdicke der Küvette [1 cm] |

Angegeben wird die Aktivität in Units (U/mL bzw. U/g), definiert als 1 µmol Substratumsatz pro Minute.

Proteinbestimmung nach Bradford:

Die Bestimmung des Proteingehaltes im Überstand wurde nach der Methode von Bradford (Anal. Chem. 1976,

72, 248-254) durchgeführt, welche auf der Bindung des Triarylmethan-Farbstoffes Coomassie Brilliant Blue G-250 an basische und aromatische Aminosäurereste im Protein basiert. Diese Bindung verursacht eine Verschiebung des Absorptionsmaximums von 465 nm auf 595 nm. Zum Erstellen der Kalibrierung wurden die Extinktionen von BSA in den Konzentrationen von 5-20 μg/L bestimmt. Hierzu wurden die jeweiligen Proben mit $H_2O$ auf 800 μL aufgefüllt und 200 μL Bradfordreagenz (Bio Rad, München) zugegeben und bei 595 nm gemessen.

Zur Bestimmung des leaching-Verhaltens der katalytisch aktiven Partikel unter harschen Reaktionsbedingungen wurde das Protokoll um folgende Schritte erweitert:

Die Bestimmung des Proteinanteils von Novozym 435 (NZ435) erfolgte nach folgendem Schema. Die NZ435-Partikel wurden für 30 min in Acetonitril/$H_20$ (1:1, v/v) bei 45 °C schüttelnd inkubiert und anschließend aus dem Überstand Proben (bspw. 1 mL) entnommen, lyophillisiert und in $H_2O$ (ebenfalls 1 mL) resupendiert. Anschließend konnte der Proteingehalt wie oben beschrieben bestimmt werden. Die Ergebnisse sind Tabelle 1 zu entnehmen.

Tabelle 1: Messergebnisse zum unbehandelten Enzymimmobilisat

| Nativ (ohne coating) | Hydrolytische Aktivität [U/mg] | PLU [U/g] | Menge desorbiertes Protein [μgProtein/mg NZ435] | Menge desorbiertes Protein [%] |
|---|---|---|---|---|
| NZ435 | 1,05 ± 0,15 | 8000 ± 500 | 56 ± 1 | 5,6 |

Vergleichsbeispiel 1: Bestimmung der mechanischen Stabilität konventioneller Enzymimmobilisate

Zwecks Bestimmung der mechanischen Stabilitäten der Partikel wurden diese in verschiedenen hochviskosen äquimolaren Substratlösungen (beispielsweise Polyethylenglycol (Molgewicht ca. 2400) und Ölsäure) unter hohen Leistungseinträgen und bei Temperaturen > 60 °C inkubiert und anschließend die Integrität der Partikel untersucht. Unter Verwendung von NZ435 (5 Gewichts-% in Polyethylenglycol (Molgewicht ca. 2400) und Ölsäure) konnte nach 24 Stunden mit bloßem Auge die Bildung feiner Partikel entdeckt werden, zum Beispiel anhand einer deutlich auftretenden Trübung.

Vergleichsbeispiel 2: Bestimmung der Desorptionsstabilität konventioneller Enzymimmobilisate

Zwecks Bestimmung der Desorptionsstabiliät der Partikel unter harschen Reaktionsbedingungen wurden Fraktionen von 50 mg NZ435 in 20 mL MeCN/$H_20$(1:1, v/v)-Lösung bei 45 °C für 30 min. geschüttelt. Aus dem Überstand wurden definierte Proben (bspw. 1 mL) entnommen und der Proteingehalt im Überstand wie oben beschrieben bestimmt. Die Partikel wurden über einen Faltenfilter zurückgewonnen und mit 100 mL $H_20$ gewaschen und für 12 h bei 50 °C getrocknet, um anschließend nach oben beschriebenen Schema die hydrolytische Aktivität und die Syntheseaktivität in PLU zu bestimmen. Die Ergebnisse sind Tabelle 2 zu entnehmen.

Tabelle 2: Messergebnisse Vergleichsbeispiel 2

| Nativ (ohne coating) | Hydrolytische Aktivität [U/mg] | Syntheseakt. [PLU/g] | Menge desorbiertes Protein [μgProtein/mgNZ435] |
|---|---|---|---|
| NZ435 | 0* | 0* | 56 |
| * Im Rahmen der oben beschriebenen Aktivitätstests war keine Aktivität mehr quantifizierbar. | | | |

<u>Beispiel 1: Herstellung eines stabilen Enzympräparates</u>

Exemplarische Herstellung:

1 g NZ435-Partikel wurden in einer Metallschale mit 1 mL Reaktionsmischung, bestehend aus verschiedenen Zusammensetzungen von Verbindungen der allgemeinen Formeln I und II (Zusammensetzung siehe Tabelle 3, die Komponenten der allgemeinen Formeln I und II wurden nach dem Fachmann geläufigen Verfahren, wie zum Beispiel in US 7,196,153 B2 beschrieben, durch Equilibrierung hergestellt), sowie Karstedt-Katalysator (Syloff 4000, Produkt von Dow Corning, USA), versetzt. Die Siliconkomponenten inklusive des Katalysators wurden jeweils vor der Applikation in 3 mL Cyclohexan gelöst und dann in die Metallschale zu den Partikeln gegeben. Nach der Zugabe wurde unmittelbar mittels Vortexer (Ika, Stufe 9) für 15-30 min. stark dispergiert bis ein Gros des Cyclohexans abgedampft war. Anschließend

wurden die Partikel für etwa 12 h im Trockenschrank bei 50 °C getrocknet.

Tabelle 3: Zusammensetzung der verschiedenen beschichteten Partikel.

| Nr. | Einwaage NZ435 | Komponente der allg. Formel I; c = d = 0; R1 = R2a = Methyl, R2b = H | Komponente der allg. Formel II; b = c = d = 0; R4 = Methyl, R5 = Vinyl | Anteil NZ435 [%] | Hydrolyseakt. [U/mg Immob. (U/mg NZ435)] | Syntheseakt. [PLU/g Immob. (PLU/g NZ435)] |
|---|---|---|---|---|---|---|
| NZ435[1] | 1 g | - | - | 100 | 1,05 | 8000 (8000) |
| i | 1 g | a = 43, b = 5 100 µL | a = 98 900 µL | 57,4 | 0,44 (0,77) | 4300 (7500) |
| ii | 1 g | a = 64,5, b = 3,5 300 µL | a = 98 700 µL | 57 | 0,39 (0,68) | 3100 (5400) |
| iii | 1 g | a = 43, b = 5 100 µL | a = 348 900 µL | 57,4 | 0,44 (0,77) | 3800 (6700) |

[1]Daten für unbehandeltes, natives Immobilisat, siehe Vergleichsbeispiel 1

Die nach diesem Verfahren hergestellten Partikel weisen gegenüber dem unbehandelten Immobilisat Aktivitätsausbeuten in der Hydrolyse von bis zu 73% (Beispiele 1 i und 1 iii, 0,77 U/mg NZ435 im Vergleich zu 1,05 U/mg für unbehandeltes NZ435, wie in Vergleichsbeispiel 1 beschrieben), bzw. 65% (Beispiel 1 i, 0,68 U/mg NZ435) auf. In der Synthese konnten Aktivitätsausbeuten von 94% (Beispiel 1 i), 84% (Beispiel 1 iii) oder 68% (Beispiel 1 ii) erreicht werden.

Beispiel 2: Bestimmung der mechanischen Stabilität erfindungsgemäßer Enzympräparate

300 mg Partikel (unbehandeltes, natives NZ435 bzw. NZ435, beschichtet gemäß iii in Tabelle 3) wurden jeweils für 90 min in 5 mL Laurinsäure bei 60 °C stark gerührt (Magnetrührplatte der Fa. Ika Modell RT Power, Stufe 5, Rührfisch: Länge 3,1 cm, Breite 0,6 cm). Nach Entfernen des Rührers wurden Fotographien der gerührten Suspensionen erstellt (Fig. 1 und 2).

Der Abbildung Fig. 1 kann deutlich entnommen werden, dass die gerührte Suspension basierend auf unbehandeltem NZ435 von feinen Partikeln getrübt ist. Die gerührte Suspension basierend auf erfindungsgemäß behandeltem NZ435 ist dagegen klar, weist also keine Partikel bzw. zumindest keine Partikel in einer sichtbaren Größe auf (Fig. 2).

Anschließend wurden die Partikel aus den Suspensionen über einen Faltenfilter abgetrennt, mit ca. 10 mL Aceton gespült und für 12 h bei 50 °C getrocknet.

Zwecks Bestimmung der mechanischen Stabilität der Partikel wurde eine Bestimmung der Korngrößenverteilung durchgeführt. Die hierbei verwendeten Siebfraktionen hatten folgende Ausschlussgrößen: 800 µm, 500 µm, 300 µm, 150 µm und 75 µm. Per Siebung wurden die Korngrößenverteilungen vor und nach dem Rühren ermittelt. Die Ergebnisse können Tabelle 4 entnommen werden.

Tabelle 4: Mittlere Korngrößenverteilung (KVT) von unbeschichtetem NZ435 und beschichtetem NZ435.

| | Komponente der allg. Formel I; c = d = 0; R1 = R2a = Methyl, R2b = H | allgemeinen Formel II; b = c = d = 0; R4 = Methyl, R5 = Vinyl | Anteil natives NZ435 [% w/w] | KVT vorher [µm] | KVT nachher [µm] | Relative Abnahme KVT [%] |
|---|---|---|---|---|---|---|
| Natives NZ435 | - | - | 100 | 392 | 293 | 25 |
| NZ435 | a = 43, b = 5 | a = 98 | 57,4 | 547 | 501 | 8,5 |

(fortgesetzt)

| | Komponente der allg. Formel I; c = d = 0; R1 = R2a = Methyl, R2b = H | allgemeinen Formel II; b = c = d = 0; R4 = Methyl, R5 = Vinyl | Anteil natives NZ435 [% w/w] | KVT vorher [μm] | KVT nachher [μm] | Relative Abnahme KVT [%] |
|---|---|---|---|---|---|---|
| gecoated | 100 μL | 900 μL | | | | |
| NZ435<br><br>gecoated | a = 64,5, b = 3,5<br>300 μL | a = 98<br><br>700 μL | 57 | 486 | 458 | 5,8 |
| NZ435<br>gecoated | A = 43, b = 5<br>100 μL | a = 348<br>900 μL | 57,4 | 491 | 432 | 12 |

Beispiel 3: Bestimmung der Desorptionsstabilität stabiler Enzympräparate

Analog zu Vergleichsbeispiel 2 wurden die gewonnenen Partikel aus Beispiel 1 mit Wasser/Acetonitril behandelt und anschließend die Hydrolyseaktivität, die Syntheseaktivität und die Menge an freigesetztem Protein bestimmt. Das Ergebnis dieser Bestimmung kann Tabelle 5 entnommen werden.

Tabelle 5: Ergebnisse aus Beispiel 3

| Probe | Hydrolyseakt. [U/mg Immob. (U/mg NZ435)] | Syntheseakt. [PLU/g Immob. (PLU/g NZ435)] | Menge desorbiertes Protein [μg/mg Immob. (μg/mg NZ435)] |
|---|---|---|---|
| NZ435 nativ (Vergleichsbeispiel 1) | 1,05 (1,05) | 8000 (8000) | - |
| NZ435 unbeschichtet (Vergleichsbeispiel 2) | 0 | 0 | 56 |
| i | 0,25<br>(0,44) | 1182 ± 13<br>(2059) | 10<br>(17) |
| ii | 0,29<br>(0,51) | 1699 ± 24<br>(2980) | 4,5<br>(7,9) |
| iii | 0,28<br>(0,49) | 1235 ± 54,3<br>(2152) | 9<br>(16) |

Während unbehandeltes, natives Enzymimmobilisat nach Inkubation keinerlei Hydrolyseaktivität zeigt und 56 μg/mg Immobilisat als freies Protein nachgewiesen werden können, zeigen die siliconbeschichteten Partikel Hydrolyseaktivitäten von bis zu 75% der Ausgangaktivität (Probe ii, 0,51 U/mg NZ435 vs. 0,77 U/mg NZ435 vor Leaching), Syntheseaktivitäten von bis zu 55 % der Ausgangsaktivität (Probe ii, 2980 U/g NZ435 vs. 5400 U/g NZ435 vor Leaching), und eine um bis zu 86% (Probe ii) reduzierte Enzymdesorption.

Beispiel 4: Herstellung eines Enzymimmobilisates

Es wurden 1 g Lewatit VPOC1600 (Fa. Lanxess) für ca. 18 h bei Raumtemperatur in 5 mL CALB-Lösung (Lipozym CALB L, Novozymes A/S, Bagsvaerd, Dänemark, hydrolytische Aktivität: 2700 U/mL) gerührt, über einen Faltenfilter abgetrennt und mit 250 mL Aqua dest. gespült, anschließend 3 h an der Luft getrocknet, mit 1 mL Isopropanol gespült und ein weiteres Mal für 1 h an der Luft getrocknet. Die so hergestellten Immobilisate wurden in verschließbaren Reaktionsgefäßen bei 4 °C bis zur weiteren Verwendung gelagert.

Nach oben beschriebenen Verfahren wurden die Aktivitäten der Enzymimmobilisate gemessen (hydrolytische Aktivität von 1,04 U/mg sowie eine Syntheseaktivität von 6000 PLU/g). Die Beladungsdichte wurde mittels Bradfordtest auf etwa 30 $\mu g_{Protein}/mg_{VPOC1600}$ bestimmt.

Die so generierten Enzymimmobilisate wurden in einem zweiten Schritt nach oben beschriebenem Verfahren mit Silicon beschichtet (Zusammensetzung so wie in Versuch i in Tab.3). Der Massenanteil an Enzymimmobilisat im Präparat entspricht 63%. Die hydrolytische Aktivität der beschichteten Präparate beträgt 0,43 U/mg und die Syntheseaktivität 2927 PLU/g.

Analog zu Vergleichsbeispiel 2 wurden die unbeschichteten sowie die mit Silicon beschichteten Partikel mit Wasser/Acetonitril behandelt und anschließend die Hydrolyseaktivität, die Syntheseaktivität und die Menge an freigesetztem Protein bestimmt. Das Ergebnis kann Tabelle 6 entnommen werden.

Tabelle 6: Ergebnisse aus Beispiel 4

| Probe | Komponente der allg. Formel I; c = d = 0; R1 = R2a = Methyl, R2b = H | Allgem. Formel II; b = c = d = 0; R4 = Methyl, R5 = Vinyl | Hydrolyseakt. [U/mg Immob. (U/mg natives Immobil.)] | Syntheseak t. [PLU/g Immob. (PLU/g natives Immobil.)] | Menge desorbiert es Protein [µg/mg Immob. (µg/mg natives Immobil.)] |
|---|---|---|---|---|---|
| CALB auf VPOC1600, unbeschich tetfrisch | - | - | 1,04 | 6000 | - |
| CALB auf VPOC1600, gecoated frisch | A = 43, b = 5<br>100 µL | a = 348<br>900 µL | 0,43<br>(0,68) | 2930<br>(4650) | - |
| CALB auf VPOC1600, unbeschich tet nach Desorpt. | - | - | 0 | 0 | 27 ± 5 |
| CALB auf VPOC1600 gecoated nach Desoprt. | A = 43, b = 5<br>100 µL | a = 348<br>900 µL | 0,234 ± 0,005<br>(0,37) | 1765 ± 369<br>(2800) | 3 ± 1,3<br>(4,7) |

Wie Tabelle 6 entnommen werden kann, wird beim unbehandelten Immobilisat die neunfache Menge an Protein desorbiert. Gleichzeitig können Aktivitätsausbeuten von 54% (Hydrolyseaktivität), bzw. 60% (Syntheseaktivität) erreicht werden. Die bereits anhand von NZ435 für käufliche Fertigpräparate gezeigte Eignung der oben beschriebenen Methode zur Beschichtung von Enzymimmobilisaten mit Siliconen konnte demzufolge auch für selbstbeladene Enzymimmobilisate nachgewiesen werden.

Beispiel 5: Bestimmung der Enzymaktivitäten in organischem Lösungsmittel

Die Aktivität der Enzympräparate aus den Beispielen 1 und 4, sowie der entsprechenden nativen Immobilisate, wird bestimmt, indem eine Propyllauratsynthese in Methylcyclohexan (Ausgangskonzentration der Substrate = 10 mM, T = 25°C) durchgeführt wird.

Tabelle 7: Syntheseaktivität (PLU/g) in Methylcyclohexan

| Nr. | Einwaage NZ435 | Komponente der allg. Formel I; c = d = 0; R1 = R2a = Methyl, R2b = H | Komponente der allg. Formel II; b = c = d = 0; R4 = Methyl, R5 = Vinyl | Anteil natives. NZ435 [% w/w] | Aktivität [U/g Immob. (U/mg natives Immob.)] | Aktivität. nach Desorption [U/g Immob. (U/g natives Immob.)] |
|---|---|---|---|---|---|---|
| NZ435 nativ | 1 g | - | - | 100 | 581 | 0 |

(fortgesetzt)

| Nr. | Einwaage NZ435 | Komponente der allg. Formel I; c = d = 0; R1 = R2a = Methyl, R2b = H | Komponente der allg. Formel II; b = c = d = 0; R4 = Methyl, R5 = Vinyl | Anteil natives. NZ435 [% w/w] | Aktivität [U/g Immob. (U/mg natives Immob.)] | Aktivität. nach Desorption [U/g Immob. (U/g natives Immob.)] |
|---|---|---|---|---|---|---|
| i aus Beispiel 1 | 1 g | a = 43, b = 5 a<br>100 µL | = 98<br>900 µL | 57,4 | 227<br>(395) | 142<br>(247) |
| ii aus Beispiel 1 | 1 g | a = 64,5, b 3,5<br>300 µL | = a = 98<br>700 µL | 57 | 240<br>(421) | 141<br>(247) |
| iii aus Beispiel 1 | 1 g | a = 43, b = 5 a<br>100 µL | = 348 x<br>900 µL | 57,4 | 267<br>(465) | 133<br>(232) |
| CALB auf VPOC1600 nativ | 1 g | - | - | 100 | 311 | 0 |
| CALB auf VPOC1600 gecoated | 1 g | a = 43, b = 5<br>100 µL | a = 98<br>900 µL | 51 | 151<br>(296) | 118<br>(231) |

Tabelle 7 zeigt deutlich, dass die Aktivitätsausbeute der Enzympräparate bei Verwendung in organischem Lösungsmittel hervorragend ist und dass die Desorptionsstabilität ebenfalls deutlich wird.

Beispiel 6: Herstellung weiterer Lipasepräparate

Analog zu Beispiel 1 wird Lipozym RM IM (Lipase aus M. miehei, immobilisiert auf Duolite A568 der Fa. Lanxess, erhältlich von Novozymes A/S) mit einem Siloxamüberzug versehen und die Aktivitätsausbeute analog zu Beispiel 5 in organischem Lösungmittel bestimmt.

Tabelle 8: Bestimmung der Aktivität von beschichteter Lipase Lipozym RM IM

| Bezeichnung | Einwaage RM IM | Komponente der allg. Formel I; c = d = 0; R1 = R2a = Methyl, R2b = H | Komponente der allg. Formel II; b = c = d = 0; R4 = Methyl, R5 = Vinyl | Anteil natives Immobil. [% w/w] | PLUs in org. LM |
|---|---|---|---|---|---|
| RM IM nativ | 1 g | - | - | 100 | 321 ± 40 |
| RM IM gecoated | 1 g | a = 43, b = 5<br>100 µL | a = 98<br>900 µL | 51 | 171 ± 5 |

Tabelle 8 zeigt deutlich, dass bezogen auf die eingesetzte Menge Lipozym RM IM im Rahmen der Messungenauigkeit eine quantitative Aktivitätsausbeute erreicht wird.

Beispiel 7: Herstellung weiterer Lipasepräparate

Analog zu Beispiel 4 wird eine Lipase aus *T. lanuginosa* (erhältlich als "Esterase" TL01 der Fa. Asa Spezialenzyme, es handelt sich um ein Lipase mit zusätzlicher Esterasefunktion) auf Lewatit VPOC1600 immobilisiert und mit einem Siloxanüberzug versehen. Zur Aktivitätsbestimmung wird die Hydrolyse Valeriansäureethylester bestimmt.

Tabelle 9: Ergebnisse aus Beispiel 5

| | Einwaage natives Immobil. | Komponente der allg. Formel I; c = d = 0; R1 = R2a = Methyl, R2b = H | allgemeinen Formel II; b = c = d = 0; R4 = Methyl, R5 = Vinyl | Anteil natives Immobil. [% w/w] | Aktivität [U/ gImmobilisat] (U/g natives Immobil.) |
|---|---|---|---|---|---|
| TL01 auf VPOC1600, nativ | 1 g | - | - | 100 | 261 |
| TL01 auf VPOC1600, gecoated | 1 g | a = 43, b = 5 250 μL | a = 198 750 μL | 55 | 157 (285) |

Tabelle 9 zeigt, dass das native Präparat eine Aktivität von 261 U/g aufweist, während das beschichtete Präparat eine Aktivität von 157 U/g zeigt. Bezogen auf den Gehalt an nativen Immobilisat bedeutet das eine Aktivität von 285 U/g, das heißt im Rahmen der Fehlergenauigkeit kann eine quantitative Aktivitätsausbeute erzielt werden.

Beispiel 8: Herstellung einer Esterasepräparation

Analog zu Beispiel 7 wird eine Esterase aus *R. oryzeae* auf Lewatit VPOC1600 immobilisiert, beschichtet und die Aktivität in der Hydrolyse von Valeriansäureethylester bestimmt.

Tabelle 10: Aktivitätsbestimmung der immobilisierten Esterase aus *R. oryzeae.*

| | Einwaage Natives Immobil. | Komponente der allg. Formel I; c = d = 0; R1 = R2a = Methyl, R2b = H | allgemeinen Formel II; b = c = d = 0; R4 = Methyl, R5 = Vinyl | Anteil natives Immobil. [% w/w] | Aktivität [U/ gImmobilisat] (U/g natives Immobil.) |
|---|---|---|---|---|---|
| Natives VPOC1600 + Esterase R.O. | 1 g | - | - | 100 | 67 |
| beschichtetes VPOC1600+E.R.O. | 1 g | a = 43, b = 5 250 μL | a = 198 750 μL | ca. 45 | 29,5 (65) |

Auch hier zeigt sich im Rahmen der Messungenauigkeit eine quantitative Aktivitätsausbeute.

Beispiel 9: Herstellung einer Laccasepräparation

Analog zu Beispiel 4 wurde eine Laccase (EC 1.10.3.2) aus *Myceliophthora Thermophilia* (erhältlich als "Flavorstar" bei der Fa. Novozymes A/S) auf Lewatit VP OC 1600 immobilisiert (4,5 mg Protein auf 1 g Lewatit VPOC 1600), mit Siloxanüberzug versehen und die Aktivität im ABTS Assay bestimmt.

Tabelle 11: Aktivitäten der immobilisierten Laccase

| Enzym/ Träger | Einwaage Natives Immobil. | Komponente der allg. Formel I; c = d = 0; R1 = R2a = Methyl, R2b = H | allgemeinen Formel II; b = c = d = 0; R4 = methyl, R5 = Vinyl | Anteil natives Immobil. [% w/w] | Aktivitat [U/g Immob. (U/g nativ. Immobil.)] | Aktivität U/g Protein | Relative Aktivität [%] |
|---|---|---|---|---|---|---|---|
| Laccase/ VPOC1600 nativ | 1 g | - | - | 100 | 0,41 | 91 | 100 |
| Laccase/ VPOC1600 gecoated | 1 g | a = 43, b = 5 250 µL | a = 98 750 µL | 33 | 0,022 (0,066) | 14,6 | 16 |

Tabelle 11 zeigt, dass sich auch Laccasen mit der erfindungsgemäßen Methode beschichten lassen.

**Patentansprüche**

1. Enzympräparate, **dadurch** erhältlich, dass Enzymimmobilisate, die Enzyme oder Enzyme enthaltende Mikroorganismen auf einen inerten Träger immobilisiert aufweisen, mit einer durch Hydrosilylierung gewonnenen Siliconbeschichtung versehen werden.

2. Enzympräparate nach Anspruch 1, **dadurch gekennzeichnet, dass** die Enzyme solche aus der Klasse der Hydrolasen, bevorzugt Lipasen, sind.

3. Enzympräparate nach einem der Ansprüche 1 bis 2, **dadurch gekennzeichnet, dass** die inerten Träger eine Korngrößenverteilung aufweisen, bei der 90% der Teilchen eine Teilchengröße von 10 bis 5000 µm aufweisen.

4. Enzympräparate nach einem der Ansprüche 1 bis 3, **dadurch gekennzeichnet, dass** die verwendeten inerten Träger aus Polyvinylstyrol, Polymethacrylat oder Polyacrylat bestehen.

5. Enzympräparate nach einem der Ansprüche 1 bis 4,
**dadurch gekennzeichnet, dass** der Siliconüberzug durch Hydrosilylierung von SiH-funktionalen Polysiloxanen mit endständigen Kohlenstoff-Kohlenstoff-Doppelbindungen enthaltenden Polysiloxanen erhalten wird.

6. Enzympräparate nach Anspruch 5, **dadurch gekennzeichnet, dass** als SiH Komponente Polysiloxane der allgemeinen

Formel I

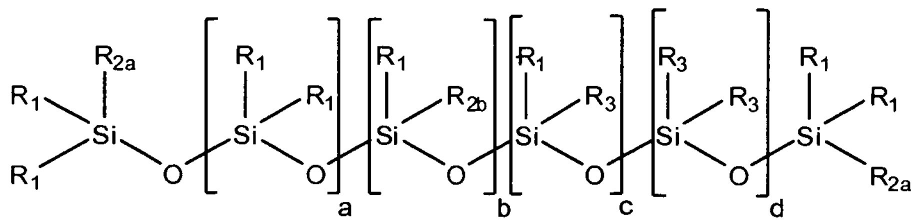

(I)

eingesetzt werden, wobei

N = a + b + c + d + 2 = 3 bis 850,

a = 1 bis 800,
b = 0 bis 400,
c = 0 bis 10,
d = 0 bis 10,
$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_{2a}$ unabhängig voneinander Wasserstoff oder R1;
$R_{2b}$ unabhängig voneinander Wasserstoff oder R1;
$R_3$ unabhängig voneinander gleiche oder verschiedene Reste der allgemeinen Formel Ia:

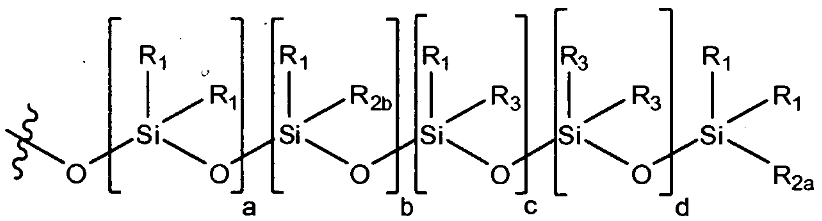

(Ia)

sind, wobei
N = a + b + c + d + 2 = 3 bis 850, vorzugsweise 6 bis 160
A = 1 bis 800,
b = 0 bis 400,
c = 0 bis 10,
d = 0 bis 10,
$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_{2a}$ unabhängig voneinander Wasserstoff oder $R_1$;
$R_{2b}$ unabhängig voneinander Wasserstoff oder $R_1$;
$R_3$ unabhängig voneinander gleiche oder verschiedene Reste der Formel Ia oder ein Rest $R_1$.

7. Enzympräparate nach Anspruch 6, **dadurch gekennzeichnet, dass** als SiH Komponente Polysiloxane der allgemeinen

Formel I

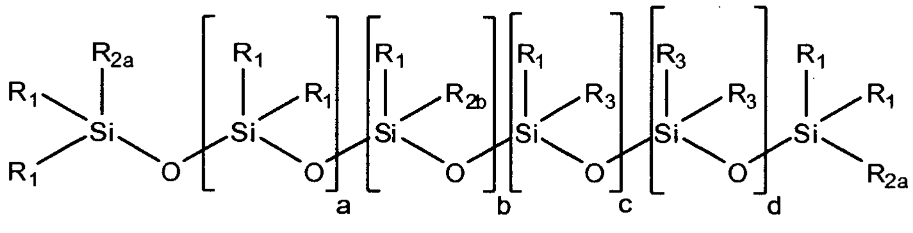

(I)

eingesetzt werden, mit

N = a + b + c + d + 2 = 6 bis 160,
a = 2 bis 150,
b = 2 bis 75
c = 0
d = 0,
$R_1$ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_{2a}$ unabhängig voneinander Wasserstoff oder $R_1$;
$R_{2b}$ unabhängig voneinander Wasserstoff oder $R_1$.

**8.** Enzympräparate nach mindestens einem der Ansprüche 5 bis 7, **dadurch gekennzeichnet, dass** als endständige Kohlenstoff-Kohlenstoff-Doppelbindung enthaltende Polysiloxane Polysiloxane der allgemeinen Formel II:

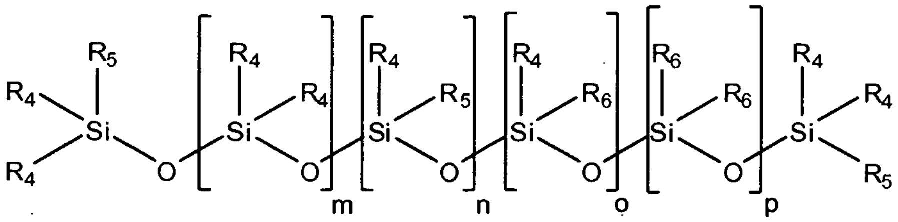

(II)

verwendet werden,
wobei

N = m + n + o + p + 2 = 3 bis 1000,
m = 1 bis 800,
n = 0 bis 20,
o = 0 bis 10,
p = 0 bis 10,
$R_4$ unabhängig voneinander gleich oder verschieden und aus folgender Gruppe sind: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_5$ unabhängig voneinander ein terminal ungesättigter Alkyl- oder Alkoxylrest oder $R_4$;
$R_6$ unabhängig voneinander gleiche oder verschiedene Reste der allgemeinen Formel IIa:

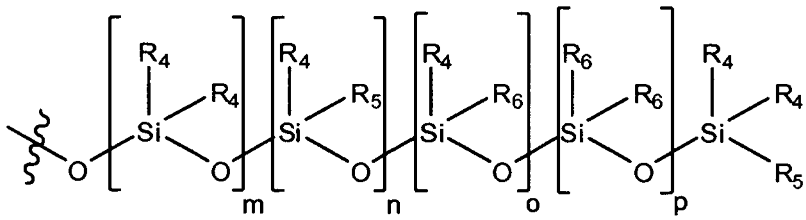

(IIa)

sind, wobei
N = m + n + o + p + 2 = 3 bis 1000,
m = 1 bis 800,
n = 0 bis 20,
o = 0 bis 10,
p = 0 bis 10,
$R_4$ unabhängig voneinander gleich oder verschieden und aus folgender Gruppe sind: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_5$ unabhängig voneinander ein terminal ungesättigter Alkyl- oder Alkoxylrest, oder $R_4$;
$R_6$ unabhängig voneinander gleiche oder verschiedene Reste der allgemeinen Formel IIa oder Reste $R_4$.

**9.** Enzympräparate nach mindestens einem der Ansprüche 5 bis 8, **dadurch gekennzeichnet, dass** als endständige Kohlenstoff-Kohlenstoff-Doppelbindung enthaltende Polysiloxane Polysiloxane der allgemeinen Formel II:

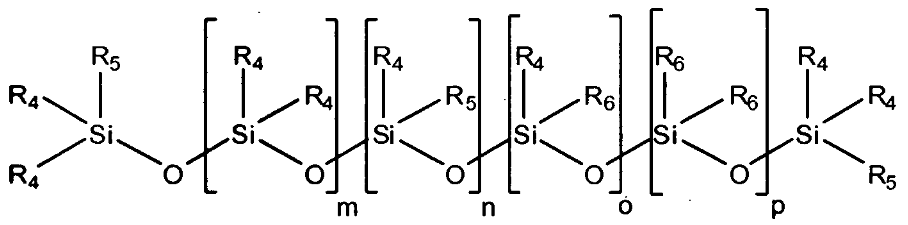

(II)

verwendet werden,
mit

N = m + n + o + p + 2 = 10 bis 600
m = 2 bis 600
n = 0 bis 10
o = 0
p = 0,
$R_4$ unabhängig voneinander gleich oder verschieden und aus folgender Gruppe sind: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen;
$R_5$ unabhängig voneinander ein terminal ungesättigter Alkyl- oder Alkoxylrest.

**10.** Verfahren zur Herstellung von Enzympräparaten nach einem der Ansprüche 1 bis 7, **dadurch gekennzeichnet, dass** Enzymimmobilisate, die Enzyme oder Enzyme enthaltende Mikroorganismen auf einen inerten Träger immobilisiert aufweisen, mit einer durch Hydrosilylierung erhaltenen Siliconbeschichtung versehen werden.

**11.** Verfahren nach Anspruch 10, **dadurch gekennzeichnet, dass** die Enzymimmobilisate **dadurch** mit einer Siliconbeschichtung versehen werden, dass die Enzymimmobilisate mit einer Reaktionsmischung, die SiHfunktionale Polysiloxane, endständige Kohlenstoff-Kohlenstoff-Doppelbindungen enthaltende Polysiloxane sowie einen die Hydrosilylierung katalysierenden Katalysator aufweist, unter Hydrosilylierungsbedingungen in Kontakt gebracht werden.

**12.** Verwendung von Enzympräparaten nach einem der Ansprüche 1 bis 9 als industrieller Biokatalysator.

## Claims

**1.** Enzyme preparations, obtainable by enzyme immobilizates which comprise enzymes or microorganisms containing enzymes immobilized on an inert carrier being provided with a silicone coating obtained by hydrosilylation.

**2.** Enzyme preparations according to Claim 1,
**characterized in that** the enzymes are those from the class of the hydrolases, preferably lipases.

**3.** Enzyme preparations according to either of Claims 1 and 2, **characterized in that** the inert carriers have a particle size distribution in which 90% of the particles have a particle size of 10 to 5000 $\mu$m.

**4.** Enzyme preparations according to any one of Claims 1 to 3, **characterized in that** the inert carriers used consist of polyvinylstyrene, polymethacrylate or polyacrylate.

**5.** Enzyme preparations according to any one of Claims 1 to 4, **characterized in that** the silicone coating is obtained by hydrosilylating SiH-functional polysiloxanes with polysiloxanes containing terminal carbon-carbon double bonds.

**6.** Enzyme preparations according to Claim 5,
**characterized in that** the SiH components used are polysiloxanes of the general formula I

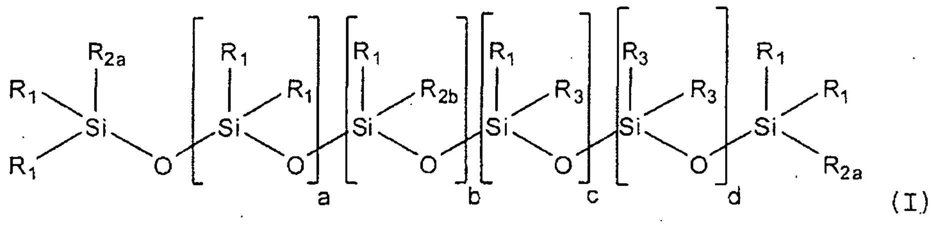

(I).

where

N = a + b + c + d + 2 = 3 to 850,
a = 1 to 800,
b = 0 to 400,
c = 0 to 10,
d = 0 to 10,
$R_1$ are independently the same or different, and are selected from the group comprising:

saturated or unsaturated, optionally branched alkyl groups having 1 to 30 carbon atoms, alkaryl radicals having 7 to 30 carbon atoms, aryl radicals having 6 to 30 carbon atoms;

$R_{2a}$ are independently hydrogen or $R_1$;
$R_{2b}$ are independently hydrogen or $R_1$;
$R_3$ are independently identical or different radicals of the general formula Ia:

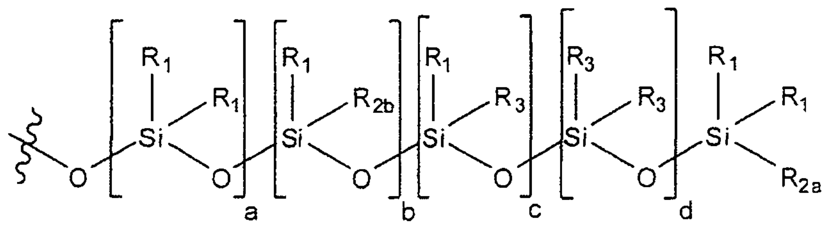

(Ia)

where
N = a + b + c + d + 2 = 3 to 850, preferably 6 to 160,
a = 1 to 800,
b = 0 to 400,
c = 0 to 10,
d = 0 to 10,
$R_1$ are independently the same or different, and are selected from the group comprising:

saturated or unsaturated, optionally branched alkyl groups having 1 to 30 carbon atoms, alkaryl radicals having 7 to 30 carbon atoms, aryl radicals having 6 to 30 carbon atoms;

$R_{2a}$ are independently hydrogen or $R_1$;
$R_{2b}$ are independently hydrogen or $R_1$;
$R_3$ are independently identical or different radicals of the formula Ia or an $R_1$ radical.

**7.** Enzyme preparations according to Claim 6,
**characterized in that** the SiH components used are polysiloxanes of the general formula I

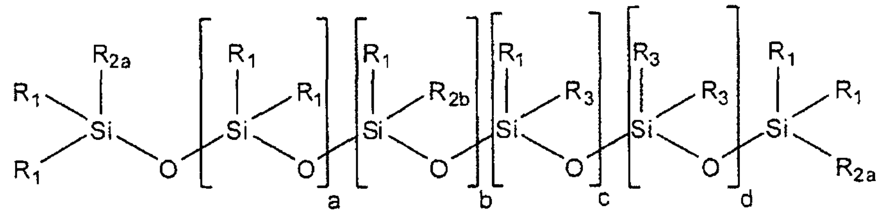

(I)

where

N = a + b + c + d + 2 = 6 to 160,
a = 2 to 150,
b = 2 to 75,
c = 0,
d = 0,
$R_1$ are independently the same or different, and are selected from the group comprising:

saturated or unsaturated, optionally branched alkyl groups having 1 to 30 carbon atoms, alkaryl radicals having 7 to 30 carbon atoms, aryl radicals having 6 to 30 carbon atoms;

$R_{2a}$ are independently hydrogen or $R_1$;
$R_{2b}$ are independently hydrogen or $R_1$.

8. Enzyme preparations according to at least one of Claims 5 to 7, **characterized in that** the polysiloxanes containing a terminal carbon-carbon double bond used are polysiloxanes of the general formula II:

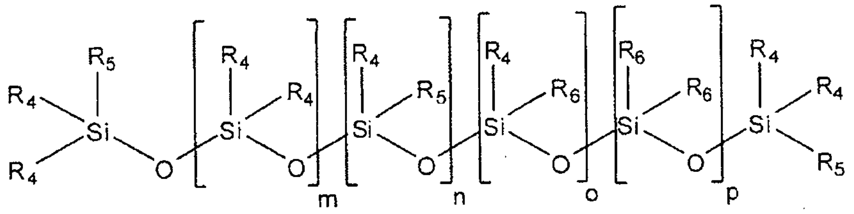

(II)

where

N = m + n + o + p + 2 = 3 to 1000,
m = 1 to 800,
n = 0 to 20,
o = 0 to 10,
p = 0 to 10,
$R_4$ are independently the same or different and are from the following group: saturated or unsaturated, optionally branched alkyl groups having 1 to 30 carbon atoms, alkaryl radicals having 7 to 30 carbon atoms, aryl radicals having 6 to 30 carbon atoms;
$R_5$ are independently a terminally unsaturated alkyl or alkoxy radical or $R_4$;
$R_6$ are independently identical or different radicals of the general formula IIa:

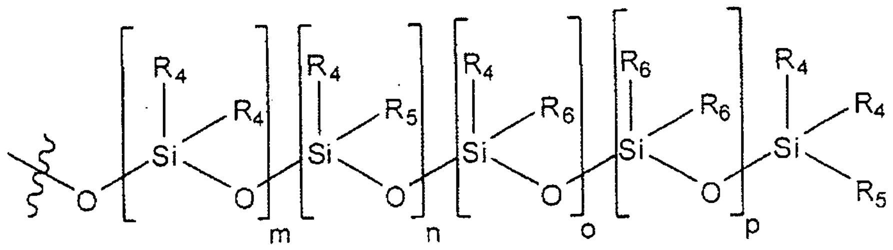

(IIa)

where
N = m + n + o + p + 2 = 3 to 1000,
m = 1 to 800,
n = 0 to 20,
o = 0 to 10,
p = 0 to 10,
$R_4$ are independently the same or different and are from the following group: saturated or unsaturated, optionally branched alkyl groups having 1 to 30 carbon atoms, alkaryl radicals having 7 to 30 carbon atoms, aryl radicals having 6 to 30 carbon atoms;
$R_5$ are independently a terminally unsaturated alkyl or alkoxy radical, or $R_4$;
$R_6$ are independently identical or different radicals of the general formula IIa or $R_4$ radicals.

**9.** Enzyme preparations according to at least one of Claims 5 to 8, **characterized in that** the polysiloxanes containing a terminal carbon-carbon double bond used are polysiloxanes of the general formula II:

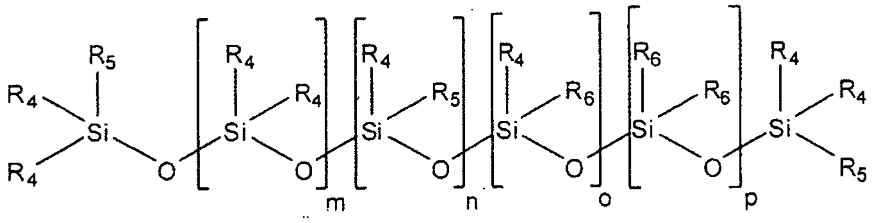

(II)

where

N = m + n + o + p + 2 = 10 to 600,
m = 2 to 600,
n = 0 to 10,
o = 0,
p = 0,
$R_4$ are independently the same or different and are from the following group: saturated or unsaturated, optionally branched alkyl groups having 1 to 30 carbon atoms, alkaryl radicals having 7 to 30 carbon atoms, aryl radicals having 6 to 30 carbon atoms;
$R_5$ are independently a terminally unsaturated alkyl or alkoxy radical.

**10.** Process for preparing enzyme preparations according to any one of Claims 1 to 7, **characterized in that** enzyme immobilizates which comprise enzymes or microorganisms comprising enzymes immobilized on an inert carrier are provided with a silicone coating obtained by hydrosilylation.

**11.** Process according to Claim 10, **characterized in that** the enzyme immobilizates are provided with a silicone coating by contacting the enzyme immobilizates with a reaction mixture which comprises SiH-functional polysiloxanes, polysiloxanes containing terminal carbon-carbon double bonds and a catalyst which catalyzes the hydrosilylation under hydrosilylation conditions.

**12.** Use of enzyme preparations according to any one of Claims 1 to 9 as an industrial biocatalyst.

## Revendications

1. Préparations d'enzymes, pouvant être obtenues par le fait que des produits d'immobilisation d'enzymes, qui comportent des enzymes ou des micro-organismes contenant des enzymes, immobilisés sur un support inerte, sont munis d'un revêtement silicone obtenu par hydrosilylation.

2. Préparations d'enzymes selon la revendication 1, **caractérisées en ce que** les enzymes sont celles appartenant à la classe des hydrolases, de préférence des lipases.

3. Préparations d'enzymes selon la revendication 1 ou 2, **caractérisées en ce que** les supports inertes présentent une distribution granulométrique dans laquelle 90 % des particules ont une taille de particule de 10 à 5 000 μm.

4. Préparations d'enzymes selon l'une quelconque des revendications 1 à 3, **caractérisées en ce que** les supports inertes utilisés consistent en polyvinylstyrène, polyméthacrylate ou polyacrylate.

5. Préparations d'enzymes selon l'une quelconque des revendications 1 à 4, **caractérisées en ce que** le revêtement silicone est obtenu par hydrosilylation de polysiloxanes à fonction SiH avec des polysiloxanes comportant des doubles liaisons carbone-carbone en bout de chaîne.

6. Préparations d'enzymes selon la revendication 5, **caractérisées en ce qu'**on utilise comme composant SiH des polysiloxanes de formule générale I

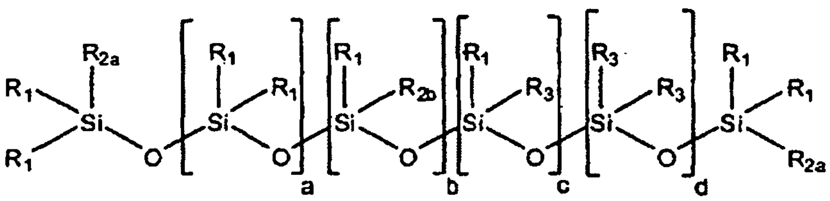

(I)

dans laquelle

N = a + b + c + d + 2 = 3 à 850,
a = 1 à 800,
b = 0 à 400,
c = 0 à 10,
d = 0 à 10,
les radicaux $R_1$, identiques ou différents, sont choisis, indépendamment les uns des autres, dans l'ensemble comprenant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone ;
$R_{2a}$ représentent, indépendamment l'un de l'autre, un atome d'hydrogène ou $R_1$ ;
$R_{2b}$ représentent, indépendamment les uns des autres, un atome d'hydrogène ou $R_1$ ;
$R_3$ représentent, indépendamment les uns des autres, des radicaux identiques ou différents de formule générale Ia :

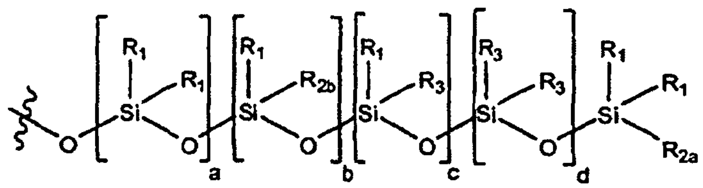

(Ia)

dans laquelle
N = a + b + c + d + 2 = 3 à 850, de préference 6 à 160,
a = 1 à 800,
b = 0 à 400,
c = 0 à 10,
d = 0 à 10,
les radicaux $R_1$, identiques ou différents, sont choisis, indépendamment les uns des autres, dans l'ensemble comprenant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone ;
$R_{2a}$ représentent indépendamment les uns des autres, un atome d'hydrogène ou $R_1$ ;
$R_{2b}$ représentent, indépendamment les uns des autres, un atome d'hydrogène ou $R_1$ ;
$R_3$ représentent, indépendamment les uns des autres, des radicaux identiques ou différents de formule Ia ou un radical $R_1$.

7. Préparations d'enzymes selon la revendication 6, **caractérisées en ce qu'**on utilise comme composant SiH des polysiloxanes de formule générale I

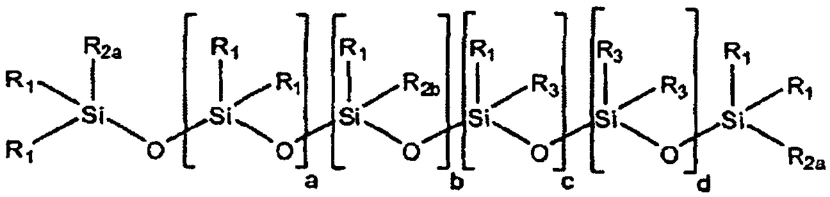

(I)

où

N = a + b + c + d + 2 = 6 à 160,
a = 2 à 150,
b = 2 à 75,
c = 0,
d = 0,
les radicaux $R_1$, identiques ou différents, sont choisis, indépendamment les uns des autres, dans l'ensemble comprenant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone ;
$R_{2a}$ représentent, indépendamment l'un de l'autre, un atome d'hydrogène ou $R_1$ ;
$R_{2b}$ représentent, indépendamment les uns des autres, un atome d'hydrogène ou $R_1$.

8. Préparations d'enzymes selon au moins l'une des revendications 5 à 7, **caractérisées en ce qu'**on utilise comme polysiloxanes comportant une double liaison carbone-carbone en bout de chaîne des polysiloxanes de formule générale II :

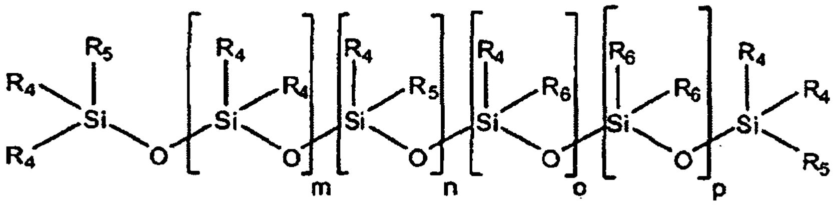

(II)

dans laquelle

N = m + n + o + p + 2 = 3 à 1000,
m = 1 à 800,
n = 0 à 20,
o = 0 à 10,
p = 0 à 10,
les radicaux $R_4$ sont identiques ou différents, indépendants les uns des autres et choisis dans l'ensemble suivant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone :
$R_5$ représentent, indépendamment les uns des autres, un radical alkyle ou alcoxy insaturé terminal ou $R_4$ ;
$R_6$ représentent, indépendamment les uns des autres, des radicaux, identiques ou différents, de formule générale IIa :

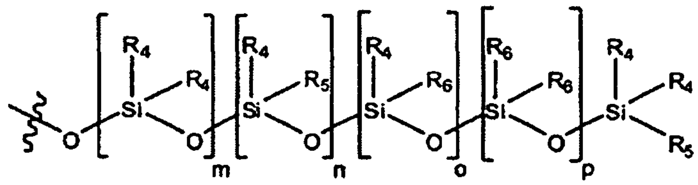

(IIa)

dans laquelle

N = m + n + o + p + 2 = 3 à 1000,
m = 1 à 800,
n = 0 à 20,
o = 0 à 10,
p = 0 à 10,
les radicaux $R_4$ sont identiques ou différents, indépendants les uns des autres et choisis dans l'ensemble suivant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone :
$R_5$ représentent, indépendamment les uns des autres, un radical alkyle ou alcoxy insaturé terminal ou $R_4$ ;
$R_6$ représentent, indépendamment les uns des autres, des radicaux, identiques ou différents, de formule générale IIa ou des radicaux $R_4$.

**9.** Préparations d'enzymes selon au moins l'une des revendications 5 à 8, **caractérisées en ce qu'**on utilise comme polysiloxanes comportant une double liaison carbone-carbone en bout de chaîne des polysiloxanes de formule générale II :

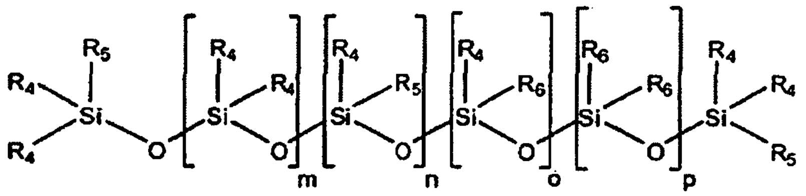

(II)

où

N = m + n + o + p + 2 = 10 à 600,
m = 2 à 600,
n = 0 à 10,
o = 0,
p = 0,
les radicaux $R_4$ sont identiques ou différents, indépendants les uns des autres et choisis dans l'ensemble suivant : des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone : $R_5$ représentent, indépendamment les uns des autres, un radical alkyle ou alcoxy insaturé terminal.

**10.** Procédé pour la production des préparations d'enzymes selon l'une quelconque des revendications 1 à 7, **caractérisé e ce que** des produits d'immobilisation d'enzymes, qui comportent des enzymes ou des micro-organismes contenant des enzymes, immobilisés sur un support inerte, sont munis d'un revêtement silicone obtenu par hydrosilylation.

**11.** Procédé selon la revendication 10, **caractérisé en ce que** les produits d'immobilisation d'enzymes sont munis d'un revêtement silicone par le fait que les produits d'immobilisation d'enzymes sont mis en contact, dans des conditions d'hydrosilylation, avec un mélange réactionnel qui comporte des polysiloxanes à fonction SiH, des polysiloxanes comportant des doubles liaisons carbone-carbone en bout de chaîne, ainsi qu'un catalyseur catalysant l'hydrosilylation.

**12.** Utilisation des préparations d'enzymes selon lune quelconque des revendications 1 à 9, en tant que biocatalyseur industriel.

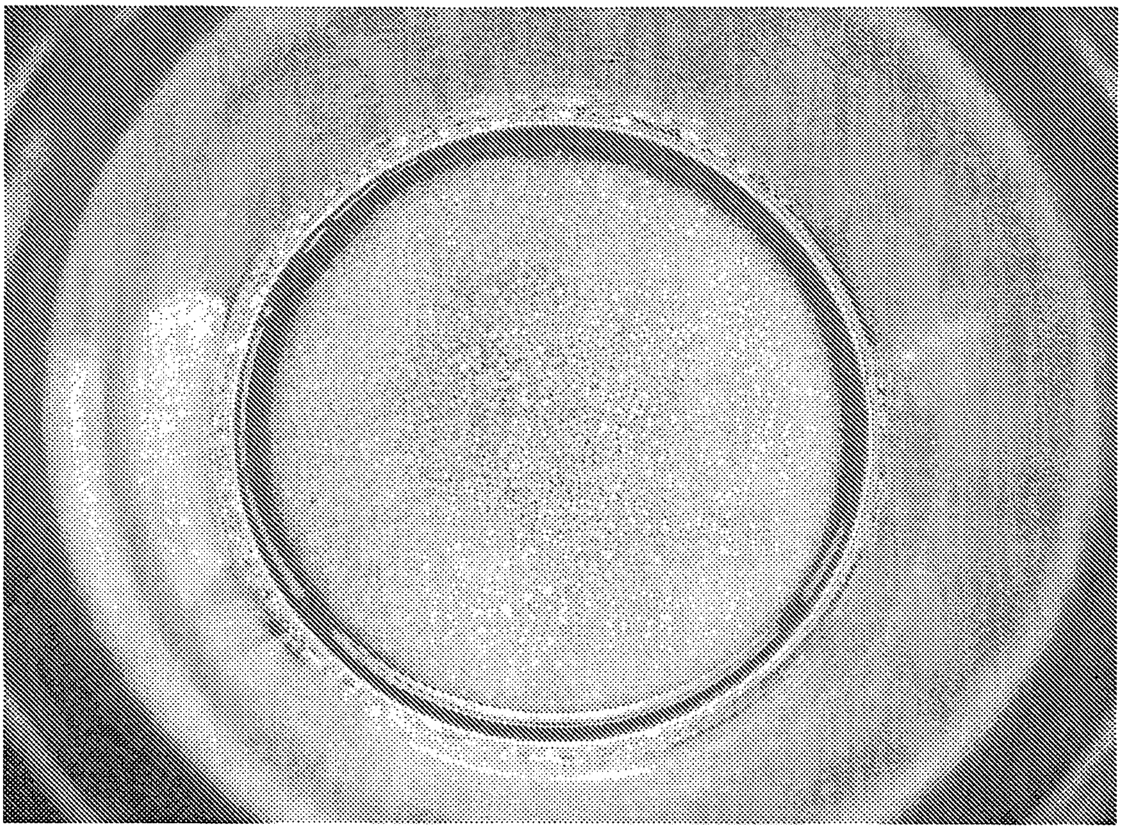

Fig. 1

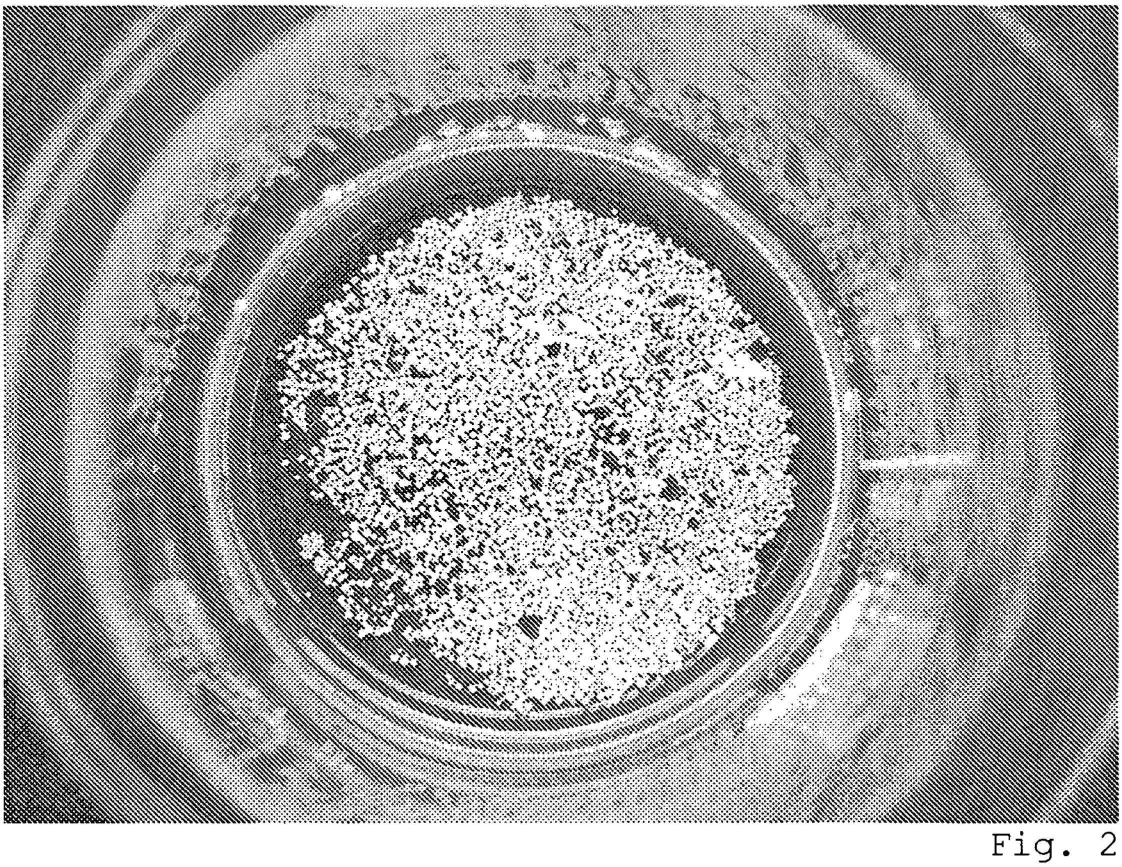

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03106607 A1
- US 7196153 B2


### In der Beschreibung aufgeführte Nicht-Patentliteratur


- **A. LIESE ; K. SEELBACH ; C. WANDREY.** Industrial Biotransformations. Wiley-VCH, 2000
- *J. Chem. Soc., Chem. Comm.,* 1989, 934-935
- *Eur. J. Lipid Sci. Technol.,* 2003, vol. 105, 601-607
- **K. FABER.** Biotransformations in Organic Chemistry. Springer, 2000, 384ff
- *J. Am. Chem. Soc.,* 1999, vol. 121, 9487-9496
- *Nippon Sanso Giho,* 1990, vol. 9, 68-72
- *Archives of Toxicology,* 1994, vol. 68, 277-283
- *Fundamental and Applied Toxicology,* 1989, vol. 13, 285-295
- *Polimery w Medycynie,* 2000, vol. 30, 45-54
- *J. Sol Gel Sci. Technol.,* 2003, vol. 26, 1183-1187
- **SONDERHEFT.** *Landbauforschung Völkenrode,* 2002, vol. 241, 41-46
- *J. Mol. Catal. B,* 2005, vol. 35, 93-99
- **L. CAO.** Carrier-bound Immobilized Enzymes: Principles, Application and Design. Wiley-VCH, 2005
- **BRADFORD.** *Anal. Chem.,* 1976, vol. 72, 248-254